(19) Europäisches Patentamt
European Patent Office
Office européen des brevets

(11) **EP 1 690 887 B1**

(12) **EUROPEAN PATENT SPECIFICATION**

(45) Date of publication and mention
of the grant of the patent:
**21.05.2008 Bulletin 2008/21**

(51) Int Cl.:
*C08J 3/12* (2006.01)     *A61L 15/60* (2006.01)
*B01J 20/26* (2006.01)

(21) Application number: **06002737.2**

(22) Date of filing: **10.02.2006**

(54) **Water absorbent resin and method for production thereof**

Wasser absorbierendes Harz und Verfahren zu seiner Herstellung

Résine absorbant l'eau et procédé de production de cette résine

(84) Designated Contracting States:
**BE DE**

(30) Priority: **15.02.2005 JP 2005038123**
**25.03.2005 JP 2005089307**

(43) Date of publication of application:
**16.08.2006 Bulletin 2006/33**

(73) Proprietor: **NIPPON SHOKUBAI CO., LTD.**
**Osaka-shi, Osaka 541-0053 (JP)**

(72) Inventors:
• **Dairoku, Yorimichi**
**Himeji-shi**
**Hyogo 672-8085 (JP)**
• **Fujino, Shin-ichi**
**Himeji-shi**
**Hyogo 671-1242 (JP)**
• **Imura, Motohiro**
**Akashi-shi**
**Hyogo 673-0891 (JP)**
• **Kato, Seiji**
**Himeji-shi**
**Hyogo 671-1242 (JP)**

(74) Representative: **Glawe, Delfs, Moll**
**Patentanwälte**
**Postfach 26 01 62**
**80058 München (DE)**

(56) References cited:
WO-A-20/05012406     US-A- 4 970 267
US-A1- 2004 068 057     US-A1- 2004 249 120
US-B1- 6 458 921

**Description**

BACKGROUND OF THE INVENTION

FIELD OF THE INVENTION

The present invention relates to a water absorbent resin and a method for production of a water absorbent resin. Particularly, the present invention relates to a water absorbent resin and a method for production of a water absorbent resin which can achieve a balance between decrease of the fine powder generated in the producing step of the water absorbent resin, and lowering of the amount of residual monomer in the resulting water absorbent resin.

Description of the Related Art

In recent years, as component materials in sanitary goods such as a disposable diaper, a sanitary napkin, an incontinence pad, and the like, water absorbent resins aiming at absorption of body fluids have been widely utilized. As the water absorbent resin, for example, cross-linked polymers of partially neutralized polyacrylic acid, hydrolysates of starch-acrylic acid graft polymer, saponified polymers of vinyl acetate-acrylic acid ester copolymers, hydrolysates of acrylonitrile copolymers or acrylamide copolymers, or cross-linked polymers thereof, cross-linked polymers of cationic monomers, and the like have been known. They may be used in the form of sheet-like, fibrous, film-like or the like, however, they have been generally used in powdery (particulate) forms. As the powder (particle), for example, those having weight average particle diameter (a mass median particle size) of approximately 200 to 800 μm have been commonly used.

Characteristics desired for such water absorbent resins involve not only water-absorption properties (absorption capacity without load, absorption capacity under a load, liquid permeability under a load and the like) but also the amount of fine powder (for example, fine particles of not greater than 150 μm) and residual monomer being small. More specifically, the fine powder may be a factor to cause decrease in liquid permeability due to clogging in absorbing articles such as diapers, and also is likely to reduce the surface crosslinking effect of the water absorbent resin (become hard to improve various physical properties such as absorption capacity under a load and the like even though it is subjected to surface crosslinking). Furthermore, it may lead to problems of the loss as well as deterioration of the working environment resulting from the dust because it is apt to fly when it is incorporated in the absorbing article such as diapers and the like. On the other hand, the amount of residual monomer is desirably as small as possible in light of the safety and odor.

Therefore, as means for reducing the fine powder upon production of the water absorbent resin, elimination of the fine powder through classification with a mesh sieve, airflow or the like has been carried out. However, the fine powder eliminated in the classification step may account for from several % to ten and several %, or may be a great deal as much as several tens %, as the case may be, of the quantity of solid content in the polymer gel obtained in the polymerization step. Therefore, to discard such fine powder may result in lowering of the yield, leading to the need of costs for discarding the fine powder. Consequently, production costs of the water absorbent resin may be elevated. Use and distribution of the eliminated fine powder, without discarding the same, for other applications in the form of the fine powder as it is may be suggested, however, the water absorbent resins in the form of the fine powder have inferior physical properties, and are accompanied by diminished demand, in general.

Hence, as a method for obtaining an inexpensive water absorbent resin with less amount of fine powder, methods in which fine powder is reutilized, i.e., fine powder recycling processes have been proposed in large numbers. Examples of the fine powder recycling process include: (I) a process in which the fine powder directly mixed with an aqueous monomer solution before the polymerization to allow for polymerization (see, United States Patent No. 5342899) ; (II) a process in which the fine powder directly mixed with a gel in the course of polymerization to allow for polymerization (see, United States Patent No. 4970267 and United States Patent No. 4950692) ; (III) a process in which the fine powder directly mixed with a polymer gel obtained by polymerization; (IV) a process in which the fine powder is agglomerated with an aqueous fluid to give greater particles followed by mixing with a polymer gel obtained by polymerization (see, United States Patent No. 6458921), and the like. Among them, the processes (I) to (III) utilize the fine powder directly, therefore, admixing of the fine powder in a homogenous manner may be difficult, and in addition, performance deterioration may be caused through absorption of the monomer, water or the like by the fine powder. Therefore, it is believed that the process (IV) in which the fine powder is utilized following agglomeration is preferred. In the process (IV), it is usually necessary to dry the used aqueous fluid because greater particles are formed through permitting binding of the fine powder with use of water as a binder, in general. Hence, it is believed to be desired that drying of the agglomerated particles obtained from the fine powder is carried out with together the polymer gel in the drying step in the original production line, i.e., in the step for drying the polymer gel obtained by polymerization, in light of the production costs and efficiency. Also, as the application of the process (IV), a process in which the fine powder is turned into the gelled product which is not particulate but is integrated and thereafter, the resulting gelled product is crushed to form particles may be suggested. However, a large amount of water may be included in gelation according to the process, therefore, it cannot be recognized as a preferable process because considerable energy is required during drying, which may elevate the production costs.

On the other hand, known means for reducing the residual monomer upon production of the water absorbent resin involve: (i) a process in which the amount of a radical polymerization initiator added to the aqueous monomer solution is increased in polymerization; (ii) a process in which a radical polymerization initiator is added to the polymer gel obtained during or following the polymerization (see, JP-B No. 63-7203 and JP-A No. 2004-517179), and the like. Among these, control of the polymerization may be difficult in the process (i), and deterioration of physical properties such as increase in extractables may be caused. Therefore, it is believed that the process (ii) may be desirable.

US 2004/0068057 A1 discloses a method for the preparation of water-absorbent resin particles comprising the steps of a polymer gel, adding an aqueous fluid containing persulfate as a thermal initiator and an oxidizing agent to the polymer gel and drying the mixture. The aqueous fluid is used in an amount of 900 g for 100 g of fines.

SUMMARY OF THE INVENTION

However, in an attempt to recycle the fine powder through agglomeration which was generated during the production steps of a water absorbent resin according to the aforementioned process (IV), the present inventor tried to dry the agglomerated particles obtained from the fine powder, together with the polymer gel after mixing them. Hence, a novel event was found that the amount of the residual monomer present in the dried matter after drying (amount of the water absorbent resin per unit weight) becomes greater than the theoretical value. In addition, even though lowering of the residual monomer is intended by adopting the aforementioned process (ii) in the aforementioned recycling process of the fine powder in the aforementioned process (IV), and adding a radical polymerization initiator such as persulfate to the polymer gel in order to solve this problem of the residual monomer, a problem of remarkable inhibition of reducing effect of the residual monomer was found.

Moreover, when the using amount of the radical polymerization initiator added to the polymer gel is increased taking into account of inhibition of reducing effect of the residual monomer by mixing the polymer with the agglomerated particles obtained from the fine powder, and then drying them together, the amount of the residual monomer in the finally resulting water absorbent resin can be reduced to a desired level. However, when a large amount of the radical polymerization initiator is used, another problem such as occurrence of coloring of the water absorbent resin, or deterioration of the physical properties may be caused.

Hence, under the current circumstances, in the production of water absorbent resins, to achieve a balance between decrease of the fine powder generated in the producing step and lowering of the amount of residual monomer in the resulting water absorbent resin has been difficult, and has not been accomplished.

Accordingly, a problem that the invention is to solve is to provide a water absorbent resin and a method for production of a water absorbent resin which can achieve a balance between decrease of the fine powder generated in the producing step and lowering of the amount of residual monomer in the resulting water absorbent resin.

The present inventor elaborately investigated to solve the problem described above. As a consequence, when the radical polymerization initiator is not added to the polymer gel as in the foregoing prior arts, but a thermal initiator (thermally decomposable radical polymerization initiator) is added to the side of the agglomerated particle obtained from the fine powder generated in the production steps of the water absorbent resin, followed by drying while allowing the resulting agglomerated particles and the polymer gel to coexist, it was found that in comparison with the case in which the thermal initiator is directly added to the polymer gel, the residual monomer can be effectively reduced by adding the same amount (per unit weight of the finally resulting water absorbent resin) thereof. Furthermore, it was also found that such an effect can be similarly yielded when an oxidizing agent or a reducing agent is used in place of the aforementioned thermal initiator. The present invention was accomplished in light of these findings.

Accordingly, the method for production of a water absorbent resin according to the present invention comprises the steps as defined in claim 1.

The water absorbent resin according to the present invention is a water absorbent resin which comprises a polymer gel having a water absorbing property and an agglomerated gel, wherein the agglomerated gel consists of fine powders having a weight average particle diameter falling within the range of from 10 to 150 $\mu$m; ratio (A/B) of the solid content rate A (%) of the agglomerated gel to the solid content rate B (%) of the polymer gel is not less than 1/3 but not greater than 3, and the amount of residual monomer is not lower than 0 but not higher than 500 ppm. The method for production of this water absorbent resin is not limited. The method for production herein disclosed encompasses an example of the method for production of this water absorbent resin.

DESCRIPTION OF THE PREFERRED EMBODIMENTS

Hereinafter, the method for production of a water absorbent resin and the water absorbent resin according to the present invention will be explained in detail, however, the scope of the present invention is not bound by these descriptions. In addition to the following exemplifications, alteration can be made ad libitum without departing from the principles of the present invention.

The method for production of the present invention comprises a step of obtaining a polymer gel having a water absorbing property (hereinafter, may be also referred to as "polymerization step"). The following steps are not limited, but in general, a step of drying the polymer gel (hereinafter, may be also referred to as "drying step"), and a step of pulverizing the dried matter of the polymer gel (hereinafter, may be also referred to as "pulverizing step"), and a step of classifying the pulverized matter (hereinafter, may be also referred to as "classification step") may be further included. Particularly, the method for production of the present invention can be referred to as being a method suited for continuously producing a water absorbent resin in which a water absorbent resin with less amount of residual monomer can be obtained through reutilizing the fine powder obtained in the production of a water absorbent resin (for example, the fine powder eliminated as a waste material in the aforementioned classification step and the like).

More specifically, in the method for production of the present invention, it is important to add an aqueous fluid containing at least one additive selected from the group consisting of thermal initiators, oxidizing agents and reducing agents (hereinafter, may be also referred to as "essential additive") to the fine powder which is obtained in the production of a water absorbent resin and has a weight average particle diameter falling within the range of 10 to 150 $\mu$m, thereby obtaining an agglomerated gel (hereinafter, may be also referred to as "agglomerated gel" or "agglomerated particle"), and to dry the agglomerated gel and the polymer gel while allowing them to coexist. In other words, the agglomerated particles obtained by mixing the fine powder and the aqueous fluid are subjected to a drying step together with the polymer gel obtained in the polymerization step.

The aforementioned agglomerated particle referred to herein may be particles that include multiple fine powders, and has a median particle diameter of the agglomerated particle being not greater than 20 mm, preferably 0.3 to 10 mm, and more preferably 0.35 to 5 mm. Therefore, when a great mass of integrated gel is obtained by mixing the fine powder and the aqueous fluid, further drying and pulverization will be necessary.

The aforementioned agglomerated particle has a moisture content of preferably not greater than 75% by weight, more preferably not greater than 70% by weight, and still more preferably not greater than 65% by weight in light of the load in drying (the lower limit being beyond 0% by weight, and preferably not less than 5% by weight). When the moisture content of the agglomerated particle becomes excessively higher than that of the polymer gel, partially incomplete drying may be perfected in drying with the polymer gel. According to the findings acquired by the present inventor, when the agglomerated particles having a low moisture content are dried together with the polymer gel in the prior arts, the amount of residual monomer of the resulting water absorbent resin tends to be more markedly increased, therefore, it was necessary to keep the moisture content of the agglomerated particle high, in light of the residual monomer even though a load may be posed in the following drying step. However, according to the present invention, the residual monomer can be reduced enough even though the moisture content is comparatively lowered, and applications of the present invention may be even more significant when the moisture content falls within the aforementioned range.

The fine powder has a particle size that is smaller than the particle size of the water absorbent resin to be obtained by the method for production of the present invention, and has been conventionally treated as a waste material, in general, as described above. Generally, it is preferred that the water absorbent resin has a weight average particle diameter (D50) (specified by JIS standard sieve classification) of 200 to 800 $\mu$m, and for example, the water absorbent resin obtained by the method for production of the present invention preferably has a weight average particle diameter (D50) falling within the range described later (200 to 450 $\mu$m). The fine powder is a residual matter yielded by excluding so that the resulting water absorbent resin has a weight average particle diameter (D50) falling within the desired range described above, and specifically, the weight average particle diameter (D50) falls within the range of 10 to 150 $\mu$m as described above. It is desired that the particles having a particle diameter of substantially less than 150 $\mu$m (specified by JIS standard sieve classification) are included in an amount of preferably 70 to 100% by weight, and still preferably 90 to 100% by weight. In addition, it is more preferred that the shape of the fine powder is amorphous obtained by aqueous polymerization than spherical obtained by inverse suspension polymerization, in light of the strength of agglomeration. Moreover, as described later, the fine powder may or may not be one subjected to a surface crosslinking treatment which has been generally performed in production of water absorbent resins, or alternatively, any mixture thereof is also permitted.

All fine powder obtained by the production of the water absorbent resin can be subjected to the aforementioned agglomeration. In general, the fine powder obtained in the classification step may be predominantly used, however, not only the fine powder obtained in the classification step but, for example, the fine powder excluded by a bag filter or the like in the production step may be used in the agglomeration. Otherwise, fine powder obtained in a different step, or fine powder obtained in a separate production process (other production apparatus) can be also mixed and used. Furthermore, the fine powder may have the same composition as that of the polymer gel to be dried together, or may have a different composition. However, preferably, the fine powder having the same composition as that derived from the polymer gel to be dried together may be used.

In light of the mixing performance with the aqueous fluid and drying efficiency, the temperature of the fine powder is preferably not lower than 35°C, more preferably 40 to 100°C, and yet more preferably 45 to 80°C. The temperature of the fine powder may be adjusted ad libitum by incubating, heating, cooling or the like in each step of the production of the water absorbent resin.

The aqueous fluid which may be used in agglomerating the fine powder is prepared by dissolving the aforementioned essential additives in a solvent. The solvent is not particularly limited, but examples thereof include e.g., water, aqueous solutions including a hydrophilic organic solvent (for example, lower alcohols such as methyl alcohol, ethyl alcohol, n-propyl alcohol, isopropyl alcohol, n-butyl alcohol, isobutyl alcohol and t-butyl alcohol; ketones such as acetone; ethers such as dioxane and tetrahydrofuran; amides such as N,N-dimethylformamide; sulfoxides such as dimethylsulfoxide, and the like), and the like. In light of the physical properties and strength of agglomeration, the solvent includes water in the range of preferably 90 to 100% by weight, more preferably 99 to 100% by weight, and the solvent including water alone is particularly preferred. Moreover, in the aqueous fluid may be also included a small amount of other additive such as a crosslinking agent, a chelating agent, a surfactant and the like in the range not to impair the advantages of the present invention. For example, as the crosslinking agent, any type of the surface crosslinking agent described later may be used. By including the crosslinking agent in the aqueous fluid, lowering of the water soluble components and improvement of the strength of agglomeration can be expected.

Although the thermal initiator which may be used as the essential additive included in the aqueous fluid is not particularly limited as long as it can be decomposed to react with the monomer when the agglomerated particles and the polymer gel are allowed to coexist and dried, but examples thereof include e.g., potassium persulfate, ammonium persulfate, sodium persulfate, t-butylhydroperoxide, hydrogen peroxide, 2,2'-azobis(2-amidinopropane) dihydrochloride and the like. Among these, peroxide is preferred, and persulfate such as sodium persulfate is particularly preferred. These thermal initiators may be used alone, or two or more thereof may be also used.

Although the oxidizing agent which may be used as the essential additive included in the aqueous fluid is not particularly limited as long as it can react with the monomer when the agglomerated particles and the polymer gel are allowed to coexist and dried, but examples thereof include e.g., inorganic oxidizing agents such as chlorate, bromate, chlorite and inorganic peroxides or organic peroxides illustrated also as the aforementioned thermal initiator such as hypochlorite, persulfate and hydrogen peroxide as well as t-butyl peroxide and benzoyl peroxide, and the like. Among these, persulfate and hydrogen peroxide are preferred, and persulfate is particularly preferred. These oxidizing agents may be used alone, or two or more thereof may be also used.

Although the reducing agent which may be used as the essential additive included in the aqueous fluid is not particularly limited as long as it can react with the monomer when the agglomerated particles and the polymer gel are allowed to coexist and dried, which may be either an organic reducing agent or an inorganic reducing agent, but may be preferably an inorganic reducing agent, and in particular, a sulfur-based, phosphorus-based, or nitrogen-based reducing agent is suitable. Specific, examples include e.g., sulfite (for example, sodium sulfite, potassium sulfite, ammonium sulfite and the like), bisulfite (for example, sodium bisulfite, potassium bisulfite, ammonium bisulfite and the like), pyrosulfite, dithionite, trithionate, tetrathionate, thiosulfate, nitrite, dimethylsulfoxide, thiourea dioxide, phosphite, nitrogen-containing organic compounds such as amino acids and ethanolamine, and the like. Among these, sulfur-based reducing agents, in particular, sulfite, bisulfite, pyrosulfite and dithionite are preferred. Preferable examples of the salt thereof include sodium salts, potassium salts and ammonium salts. Among all, sodium sulfite and sodium bisulfite are particularly preferred. These reducing agents may be used alone, or two or more thereof may be also used.

As the essential additive, a thermal initiator is preferred among those described above, and in particular, use of persulfate as being essential one is preferred in light of possible achievement of excellent effect of reducing the residual monomer.

Content of the essential additive in the aqueous fluid is not particularly limited, but is preferably 0.0001 to 1% by weight per the fine powder, in general. When the content is less than 0.0001% by weight, the residual monomer may not be reduced enough, while in contrast, when it is beyond 1% by weight, coloring of finally resulting water absorbent resin after drying may be caused.

In addition, the essential additive (particularly, thermal initiator) may be used in the polymerization step, as the case may be. In such instance, with respect to the content of the essential additive in the aqueous fluid, in general, the amount per unit weight of the fine powder before the agglomeration is preferably 1 to 500% by weight, more preferably 5 to 400% by weight, and still more preferably 10 to 300% by weight of the amount of the essential additive added in the polymerization step (amount per unit weight of the monomer component). When the proportion of the essential additive included in the aqueous fluid per the essential additive added in the polymerization step is out of the above range, the advantage of the present invention may be impaired. In the cases as described above (i.e., when the essential additive is used in the polymerization step), the essential additive added in the polymerization step and the essential additive included in the aqueous fluid may be the same type or the different type.

Characteristics of the method for production of the water absorbent resin according to the present invention are to include the essential additive in the aqueous fluid. Although the effect in terms of the adding amount may be low in comparison with this case, the essential additive (particularly, thermal initiator) may be added separately in an arbitrary step of the method for production of the present invention apart from the addition to the aqueous fluid, for example, addition to the polymer gel before subjecting to the drying step, addition to the dried matter (dried polymer gel) before subjecting to the pulverizing step, or the like.

Using amount of the aqueous fluid is not less than 25 parts by weight but not greater than 150 parts by weight per 100 parts by weight of the fine powder. When the using amount of the aqueous fluid is beyond 280 parts by weight, great mass of integrated gel may be obtained, which may lead to necessity of further drying and pulverizing the gelatinous matter to give the agglomerated particles, and to need of an enormous load for the drying. In contrast, when the using amount of the aqueous fluid is less than 25 parts by weight, strength of agglomeration may be insufficient, thereby possibly disabling excellent characteristics of the final product, accompanied by difficulties in agglomeration due to heterogeneous mixing.

Although mixing of the fine powder and the aqueous fluid may be merely executed upon agglomeration of the fine powder, however, in particular, it is preferred that the aqueous fluid is previously heated upon the agglomeration. Still more, according to a preferred embodiment, the heated aqueous fluid and the fine powder are mixed at a high speed, thereby permitting agglomeration. Hence, not great mass of integrated gel, but agglomerated particles the particle size of which was directly controlled can be obtained. As a consequence, further drying and pulverizing the gelatinous matter to give agglomerated particles may not be required, and in addition, problems can be avoided that are caused in obtaining the great mass of integrated gel, i.e., problems of deterioration of the water absorbent resin itself due to cleavage, entanglement or the like of the main chain can be avoided, which may result from a huge force required in mixing, or from a kneaded state of a gelatinous block being formed.

In preferred embodiments of the agglomeration, the temperature of the aqueous fluid upon heating may be usually not lower than 40°C, preferably not lower than 50°C, and more preferably not lower than 60°C, and still more preferably not lower than 70°C. Moreover, the upper limit of the temperature may be not higher than the boiling point of the aqueous fluid, and the boiling point may be adjusted diversely by alteration of addition of a salt or other solvent, pressure (decompression, compression) and the like. However, great alteration is not caused even though the temperature is higher than 100°C, therefore, the temperature of not higher than 100°C may be usually employed. When the aqueous fluid is previously heated, it is preferred that the essential additive is separately prepared to give an aqueous solution having a comparatively high concentration at room temperature or under cooling, and then mixed with the remaining aqueous fluid in a comparatively large amount which had been heated, immediately before mixing with the fine powder.

In a preferred embodiment of the agglomeration, it is preferred that the fine powder itself is also heated in addition to previously heating the aqueous fluid. The temperature of the fine powder upon heating is also not lower than 40°C, and preferably not lower than 50°C, in general. Because great alteration is not caused even though the temperature is higher than 100°C, the temperature of not higher than 100°C may be usually employed. When the fine powder itself is previously heated, the procedure is not particularly limited, but for example, the heating may be executed through incubation after heating by drying, or through heating externally in a separate manner.

In preferred embodiments of the agglomeration, upon mixing at a high speed of the heated aqueous fluid and the fine powder, mixing at a high speed means that time period required for completing mixing of the aqueous fluid and the fine powder to produce agglomerated particles is short. More specifically, time period starting from the time point at which the aqueous fluid is brought into contact with the fine powder until the time point at which the agglomerated particles are produced, i.e., mixing time, is short. The mixing time is preferably not longer than 3 min, more preferably not longer than 1 min, and most preferably from 1 sec to 60 sec. When the mixing time is long, homogenous mixing of the aqueous fluid and the fine powder may become difficult, and a great mass of integrated gel is liable to be formed. Furthermore, when the mixing time is too long, the essential additive included in the aqueous fluid may be decomposed before subjecting the generated agglomerated particles to the drying step together with the polymer gel, thereby raising impossibilities of the essential additive to be present in a sufficient amount in the drying step. Moreover, when the mixing is continued for a long period of time after completing the mixing, deterioration of performances of the resulting water absorbent resin may be caused such as increase in water extractables, lowering of the absorption capacity under a load, and the like of the water absorbent resin.

Exemplary means for accomplishing the mixing at a high speed may involve charging the heated aqueous fluid into the fine powder at once while stirring. In other words, when the aqueous fluid is gradually added by a method of, for example, spraying or the like, deterioration of the water absorbent resin may be caused because the fine powder may form a great aggregated block during the operation, or may be kneaded. Time period for charging the heated aqueous fluid is preferably not longer than 60 sec, more preferably not longer than 30 sec, and most preferably not longer than 10 sec. Also, as means for achieving the mixing at a high speed, a process in which the fine powder is charged into the heated aqueous fluid while stirring, contrary to the above process, may be also illustrated. In this instance, the time period for charging the fine powder is preferably not longer than 60 sec, more preferably not longer than 30 sec, and most preferably not longer than 10 sec. In addition, exemplary means for accomplishing the mixing at a high speed may also involve concomitantly mixing the fine powder with the heated aqueous fluid at once. In this instance, time period for charging both of them is preferably not longer than 60 sec, more preferably not longer than 30 sec, and most preferably not longer than 10 sec. Also, the agglomerated particles can be also obtained continuously through concomitantly charging both of them in continuity to allow for mixing at a high speed. As described above, the time period required until drying the agglomerated particles and the polymer gel while allowing them to coexist is preferably as short as possible, taking into account of the decomposition of the essential additive. When the polymer gel is obtained continuously in the polymerization step, the agglomerated particles are mixed therein continuously to subject to the drying step in a short time period in a preferred embodiment.

Whether the generated agglomerated matter is agglomerated particles or not may be ascertained by a optical microscope microscopy based on the fact that a plurality of individual particles flock to aggregate while keeping their shape, and the fact that they are swollen as multiple discontinuous particles in absorption of a liquid.

Upon drying the agglomerated particles and the polymer gel while allowing them to coexist, the difference between the solid content rate B (%) of the polymer gel and the solid content rate A (%) of the agglomerated particles (or, difference between the moisture content rate of the polymer gel and the moisture content rate of the agglomerated particles) is preferably as small as possible. Specifically, ratio (A/B) of the solid content rate A (%) of the agglomerated particles (agglomerated gel) to the solid content rate B (%) of the polymer gel is not less than 1/3 but not greater than 3, more preferably not less than 1/2 but not greater than 2, still more preferably not less than 2/3 but not greater than 3/2, yet more preferably not less than 4.5/5.5 but not greater than 5.5/4.5, and particularly preferably 1 (i.e., the solid content rates being substantially the same). When the ratio (A/B) of the solid content rate of the agglomerated particles to the solid content rate of the polymer gel is out of the above range, drying of both components which had been mixed is liable to be heterogeneous. Thus, either one may be overdried or undried, thereby possibly causing troubles in production and problems in quality.

The term "solid content" refers to the residue yielded by eliminating volatile components (predominantly water) from the gelatinous water absorbent resin (polymer gel or agglomerated gel), i.e., resin component of the water absorbent resin. Herein, the weight of the solid content as described above is referred to as "quantity of solid content", while the ratio of the quantity of solid content per the weight of the gelatinous water absorbent resin including volatile components is represented by "solid content rate (%)". Also, the term "moisture content" refers to the proportion (%) of water included in the gelatinous water absorbent resin, corresponding approximately to the value derived by subtracting the aforementioned solid content rate (%) from 100%.

Upon drying while allowing the agglomerated particles and the polymer gel to coexist, ratio of the agglomerated particles to the polymer gel (in other words, recycling amount of the fine powder per the quantity of solid content in the polymer gel obtained in the polymerization step) is determined so that the quantity of solid content of the agglomerated particles (agglomerated gel), i.e., the quantity of solid content of the fine powder before the agglomeration, becomes preferably not greater than 40% by weight, and still more, to be not greater than 35% by weight, not greater than 30% by weight, not greater than 25% by weight, not greater than 20% by weight, not greater than 15% by weight is preferred in due order per the quantity of solid content in the polymer gel. Additionally, any lower limit is permissible as long as it is beyond 0% by weight. In general, it is considered that the recycling amount of the fine powder per the quantity of solid content in the polymer gel obtained in the polymerization step of beyond 40% by weight is not practical in light of the production efficiency.

Taking into consideration of the recycling amount of the fine powder being at most 40% by weight, the amount of the essential additive used through adding to the aqueous fluid is very small with respect to the entire water absorbent resin obtained after drying the agglomerated particles together with the polymer gel, which is at the very most less than 0.3% by weight. When the recycling amount of the fine powder is approximately ten and several% by weight, the amount of the essential additive may be less than 0.1% by weight. In other words, the method for production of the present invention in which the essential additive is added to the aqueous fluid enables the residual monomer to be reduced to the desired level with a very small amount of the essential additive, in comparison with the cases in which lowering of the residual monomer to the desired level is intended through adding all the used essential additives to the polymer gel.

In the method for production of the water absorbent resin according to the present invention, the agglomerated particles and the polymer gel are dried while allowing them to coexist. Preferably, it is desired to execute drying in the state in which at least a part of the agglomerated particles is brought into contact with at least a part of the polymer gel. Alternatively, it is preferred that the drying is executed through mixing the agglomerated particles with the polymer gel. The drying may be executed in the state of the agglomerated particles being homogenously mixed with the polymer gel, or in the state of the agglomerated particles being slightly mixed or hardly mixed with the polymer gel. In other words, according to the method for production of the water absorbent resin of the present invention, lowering of the residual monomer can be sufficiently accomplished by merely drying in the state of being slightly mixed or hardly mixed as described above, without particularly mixing to give a homogenous state. Specifically, in connection with the polymer gel that flows in a pipe or on a conveying belt connected to an apparatus for carrying out drying, for example, all needed is to allow the agglomerated particles to converge into the pipe or to supply on the conveying belt, followed by executing drying together as they stand. According to the prior arts in which persulfate or the like is added to the polymer gel thereby reducing the residual monomer, it is important to homogeneously distribute the additive such as persulfate or the like to the overall polymer gel. However, taking into account of separate operation of mixing also becoming necessary, it is indicated that the mechanisms of reducing the residual monomer of the prior art may be different from the present invention in this respect. In other words, according to the method for production of the water absorbent resin of the present invention, it is believed that the essential additive included in the agglomerated particles may not necessarily act on lowering of the residual monomer through being mixed homogenously with the polymer gel in the form of agglomerated particles, but it may exert some effect during drying. As a matter of course, it is anyhow permissible to homogeneously mix the agglomerated particles with the polymer gel.

The method for drying and conditions thereof and the like in drying the agglomerated particles and the polymer gel while allowing them to coexist will be described in detail in Drying Step described later.

Hereinafter, the polymerization step that is essential in the method for production according to the present invention, and the drying step, the pulverizing step and the classification step which may be carried out as needed will be explained in detail.

(Polymerization Step)

The aforementioned polymerization step is a step for producing a polymer gel having a water absorbing property through polymerizing the monomer which can be a water absorbent resin by polymerization. The method for polymerization used in the method for production according to the present invention is not particularly limited, but bulk polymerization or precipitation polymerize can be carried out. However, in light of the performances and ease of controlling the polymerization, inverse suspension polymerization or aqueous polymerization in which the monomer is provided in an aqueous solution is preferred.

The monomer is not particularly limited, but the examples thereof include the following monomers, e.g., anionic unsaturated monomers such as (meth)acrylic acid, (anhydrous)maleic acid, itaconic acid, cinnamic acid, vinyl sulfonic acid, allyl toluenesulfonic acid, vinyl toluenesulfonic acid, styrenesulfonic acid, 2-(meth)acrylamide-2-methylpropanesulfonic acid, 2-(meth)acryloylethanesulfonic acid, 2-(meth)acryloylpropanesulfonic acid and 2-hydroxyethyl(meth)acryloyl phosphate, and salts thereof; mercapto group-containing unsaturated monomers; phenolic hydroxyl group-containing unsaturated monomers; amide group-containing unsaturated monomers such as (meth)acrylamide, N-ethyl(meth)acrylamide and N,N-dimethyl(meth)acrylamide; amino group-containing unsaturated monomers such as N,N-dimethylaminoethyl(meth)acrylate, N,N-dimethylaminopropyl(meth)acrylate and N,N-dimethylaminopropyl(meth)acrylamide; and the like. These monomers may be used alone, or two or more may be used as a mixture ad libitum. However, in light of the performances and cost of the resulting water absorbent resin, an acrylic acid and/or a salt thereof (for example, a salt of sodium, lithium, potassium, ammonium, amine or the like; among them, sodium salt being preferred in light of the cost) is preferably used as a principal component. Using amount of acrylic acid and/or a salt thereof is preferably not less than 70% by mole, more preferably not less than 80% by mole, still more preferably not less than 90% by mole, and particularly preferably not less than 95% by mole (the upper limit being 100% by mole) per the entire monomer component (except for the internal crosslinking agent described later). When the monomer is an acid group-containing monomer, its neutralization ratio is not particularly limited, but the neutralization may be performed following the neutralization as needed. For applications such as in sanitary goods which may be brought into contact with a human body, also taking into consideration of unnecessity of neutralization post the polymerization, the neutralization ratio may be preferably not less than 40% by mole but not greater than 90% by mole, and more preferably not less than 50% by mole but not greater than 80% by mole.

In the polymerization step, when the monomer is provided in an aqueous solution, the concentration of the monomer in the aqueous solution (hereinafter, may be also referred to as monomer solution") is not particularly limited, but may fall within preferably the range of 10 to 70% by weight, and more preferably within the range of 20 to 60% by weight. Also, when the aqueous polymerization or the inverse suspension polymerization is carried out, a solvent other than water may be used in combination as needed, and the type of the solvent which may be used in combination is not particularly limited.

In the polymerization step, a radical polymerization initiator can be used to execute polymerization. The radical polymerization initiator is not particularly limited, but one, or two or more may be selected and used among those which have been utilized in production of common water absorbent resins depending on the type of the monomer to be polymerized and polymerization conditions. Examples of the same include pyrolytic initiators (e.g., persulfate such as sodium persulfate, potassium persulfate and ammonium persulfate; peroxide such as hydrogen peroxide, t-butyl peroxide, and methylethylketone peroxide; azo compounds such as azonitrile compounds, azoamidine compounds, cyclic azoamidine compounds, azoamide compounds, alkylazo compounds, 2,2'-azobis(2-amidinopropane)dihydrochloride and 2,2'-azobis[2-(2-imidazoline-2-yl)propane]dihydrochloride; and the like), as well as photolytic initiators (e.g., benzoin derivatives, benzyl derivatives, acetophenone derivatives, benzophenone derivatives, azo compounds and the like), and the like. Among these, pyrolytic initiators are preferred, and persulfate is particularly preferred in light of the cost performances and reducing capability of the residual monomer. Also, use of a reducing agent that accelerates decomposition of these radical polymerization initiators can make a redox initiator through combining both compounds. Examples of the reducing agent include e.g., (bi) sulfurous acid (sulfite) such as sodium sulfite and sodium bisulfite, L-ascorbic acid (ascorbate), reducing metals (salts) such as ferrous salt, amines, and the like, but not particularly limited thereto. More preferably, the photolytic initiator and the pyrolytic initiator are used in combination. Using amount of the radical polymerization initiator which may be used in the polymerization step is not particularly limited, but in general, may be preferably 0.001 to 2% by weight, and more preferably 0.01 to 0.05% by weight per the monomer. The using amount of the radical polymerization initiator being less than 0.001% by weight is not preferred because the quantity of unreacted monomer may be so great that the quantity of residual monomer in the resulting water absorbent resin is increased. In contrast, the using amount thereof beyond 2% by weight is not preferred because water extractable polymer content in the resulting water absorbent resin is increased.

In the polymerization step, instead of using the radical polymerization initiator, the polymerization reaction may be carried out through irradiating an active energy ray such as a radiation ray, an electron ray, an ultraviolet ray or the like to the reaction system.

In the polymerization step, an internal crosslinking agent may be used as needed. As the internal crosslinking agent, any of conventionally known internal crosslinking agents having two or more polymerizable unsaturated groups, or two or more reactive groups in one molecule can be used. Specific examples thereof include e.g., N,N'-methylenebis (meth)acrylamide, (poly)ethylene glycol di(meth)acrylate, (poly)propylene glycol di(meth)acrylate, trimethylolpropane tri (meth)acrylate, glycerol tri(meth)acrylate, glycerol acrylate methacrylate, ethylene oxide modified trimethylolpropane tri (meth)acrylate, pentaerythritol hexa(meth)acrylate, triallyl cyanurate, triallyl isocyanurate, triallyl phosphate, triallylamine, polyallyloxyalkane, (poly)ethylene glycol diglycidyl ether, glycerol diglycidyl ether, ethylene glycol, polyethylene glycol, propylene glycol, glycerol, 1,4-butanediol, pentaerythritol, ethylenediamine, ethylene carbonate, propylene carbonate, polyethyleneimine, glycidyl (meth) acrylate and the like. One, or two or more may be used among these taking into account of the reactivity. Particularly, it is preferred that a compound having two or more polymerizable unsaturated groups is essentially used as the internal crosslinking agent. Using amount of the internal crosslinking agent may be determined ad libitum depending on the physical properties of the desired water absorbent resin, however, in general, it may fall within the range of 0.001 to 5% by mole per the monomer. When the using amount of the internal crosslinking agent is too small, the gel strength may be lowered, and the extractables tend to be increased. In contrast, when the using amount is too great, the absorption capacity tends to be deteriorated. The internal crosslinking agent may be added to the reaction system once in bulk, or in divided fractions.

In the polymerization step, any of various foaming agents such as carbonate (hydrogen) salts, carbon dioxide, azo compounds and inert organic solvents; a hydrophilic polymer such as starch cellulose, a derivative of starch cellulose, polyvinyl alcohol, polyacrylic acid (polyacrylate) or polyacrylic acid (polyacrylate) cross-linked polymer; any of various surfactants; a chain transfer agent such as hypophosphorous acid (hypophosphite) ; or the like may be added ad libitum to the reaction system as needed in the range not to impair the effect of the present invention (for example, each foaming agent may be not more than 30 parts by weight, hydrophilic polymer may be not more than 30 parts by weight, chain transfer agent may be not more than 1 part by weight, per 100 parts by weight of the monomer).

Although the polymerization temperature in the aforementioned polymerization step is not particularly limited, but may be, in general, preferably 10 to 140°C. When the polymerization temperature is less than 10°C, the polymerization time becomes so long that productivity may be reduced, and also, the physical properties of the water absorbent resin may be deteriorated. In contrast, when the polymerization temperature is beyond 140°C, the physical properties of the water absorbent resin may be deteriorated. Also, the polymerization time is not particularly limited, but may be determined ad libitum depending on type of the monomer and the polymerization initiator, as well as the polymerization temperature. Moreover, the polymerization may be usually carried out under an ordinary pressure in light of the apparatus, ease of operation and the like, however, in a preferred embodiment, it may be carried out under a reduced pressure for the purpose of lowering the boiling temperature of the polymerization system.

(Drying Step)

The drying step is a step for drying the polymer gel obtained in the polymerization step, or the agglomerated gel obtained from the polymer gel and the fine powder. It is preferred that the polymer gel obtained in the polymerization step is generally subjected to the drying step in a state of particles of approximately 0.1 to 5 mm. The method for drying in the drying step is not particularly limited, but any method in which a common dryer or heating oven is used can be universally adopted. It is preferred that the drying temperature is a comparatively high temperature, and specifically, it may be preferably 100 to 250°C, more preferably 120 to 220°C, and still more preferably 150 to 200°C. The drying time is not particularly limited, which may be determined to be a time period such that the resultant dried matter has a desired solid content rate. It is preferred that the dried matter obtained in the drying step has a solid content rate (weight loss in drying after heating at 180°C for 3 hrs) of not less than 90% by weight, in light of ease of pulverization. In general, it is preferred that the drying time is usually within 2 hours in light of the production efficiency although it may vary depending on the particle size of the polymer gel and the agglomerated gel, the drying temperature, airflow and the like.

(Pulverizing Step)

The pulverizing step is a step for pulverizing the polymer gel. Although the pulverization may be generally carried out on the dried matter of the polymer gel obtained in the drying step, it may be also carried out on the polymer gel before drying obtained in the polymerization step. The pulverization is preferably carried out so that more particles having a desired particle diameter (preferably, weight average particle diameter being 200 to 800 $\mu$m, as described above) can be obtained. Method for pulverization is not particularly limited, but any conventionally known method can be adopted.

(Classification Step)

The classification step is a step for classifying the pulverized matter obtained in the pulverizing step. In the classification step, the aimed water absorbent resin is obtained through selecting particles having a desired particle diameter (preferably, weight average particle diameter being 200 to 800 $\mu$m, as described above), and in addition, the fine powder for subjecting to the aforementioned agglomeration is obtained as a residual matter. The method for classification is not particularly limited, but any conventionally known method can be adopted.

In the method for production of the present invention, after the classification step, the resulting water absorbent resin may be preferably subjected to a step for allowing for crosslinking around the particle surface as needed. Hereinafter, the surface crosslinking step will be further explained.

(Surface Crosslinking Step)

As the surface crosslinking agent, any conventionally known surface crosslinking agent may be suitably used. Examples thereof include e.g., polyhydric alcohol compounds such as ethylene glycol, diethylene glycol, propylene glycol, triethylene glycol, tetraethylene glycol, polyethylene glycol, 1,3-propanediol, dipropylene glycol, 2,2,4-trimethyl-1,3-pentanediol, polypropylene glycol, glycerol, polyglycerol, 2-butene-1,4-diol, 1,4-butanediol, 1,5-pentanediol, 1,6-hexanediol, 1,2-cyclohexanedimethanol, 1,2-cyclohexanediol, trimethylolpropane, diethanolamine, triethanolamine, polyoxypropylene, oxyethylene-oxypropylene block copolymers, pentaerythritol and sorbitol; epoxy compounds such as ethyleneglycol diglycidyl ether, polyethyleneglycol diglycidyl ether, glycerol polyglycidyl ether, diglycerol polyglycidyl ether, polyglycerol polyglycidyl ether, propyleneglycol diglycidyl ether, polypropyleneglycol diglycidyl ether and glycidol; polyamine compounds such as ethylenediamine, diethylenetriamine, triethylenetetramine, tetraethylenepentamine, pentaethylenehexamine and polyethyleneimine, inorganic salts or organic salts (aziridinium salts and the like) thereof; polyisocyanate compounds such as 2,4-tolylene diisocyanate and hexamethylene diisocyanate; polyoxazoline compounds such as 1,2-ethylenebisoxazoline; alkylene carbonate compounds such as 1,3-dioxolane-2-one, 4-methyl-1,3-dioxolane-2-one, 4,5-dimethyl-1,3-dioxolane-2-one, 4,4-dimethyl-1,3-dioxolane-2-one, 4-ethyl-1,3-dioxolane-2-one, 4-hydroxymethyl-1,3-dioxolane-2-one, 1,3-dioxane-2-one, 4-methyl-1,3-dioxane-2-one, 4,6-dimethyl-1,3-dioxane-2-one and 1,3-dioxopane-2-one; haloepoxy compounds such as epichlorohydrin, epibromohydrin and $\alpha$-methyl epichlorohydrin; polyvalant metal compounds such as hydroxides and chlorides of zinc, calcium, magnesium, aluminum, iron, zirconium or the like; and the like. Among these surface crosslinking agents, at least one compound selected from the group consisting of polyhydric alcohol compounds, epoxy compounds, polyamine compounds and salts thereof, alkylene carbonate compounds may be suited. These surface crosslinking agents may be used alone, or two or more may be used as a mixture taking into consideration of the reactivity. The surface crosslinking step may be carried out twice or more in light of the effect of the same. In this instance, the second cycle and the followings may be carried out using the same surface crosslinking agent as that in the first cycle, but they may be carried out using a different surface crosslinking agent.

Using amount of the surface crosslinking agent falls within preferably the range of 0.001 to 10 parts by weight, and more preferably the range of 0.01 to 5 parts by weight per 100 parts by weight of the solid content of the water absorbent resin, although it may vary depending on the used compound and the combination thereof. Use of the surface crosslinking agent within this range enables crosslinking density around the surface of the water absorbent resin to be higher than that inside thereof. The using amount of the surface crosslinking agent of beyond 10 parts by weight is not preferred because of not only economical inefficiency, but also excessive amount of the necessary crosslinking agent upon forming an optimal cross-linked structure as an absorbing agent. The using amount of the surface crosslinking agent of less than 0.001 parts by weight is not preferred because its improving effect as an absorbing agent is hardly achieved in upgrading the performance such as the absorption capacity under a load or the like.

When the surface crosslinking is executed, upon mixing of the water absorbent resin and the surface crosslinking agent, water is preferably used as a solvent. Using amount of water may be not lower than 0 part by weight, preferably not greater than 20 parts by weight, and more preferably within the range of 0.5 to 10 parts by weight per 100 parts by weight of the solid content of the water absorbent resin, although it may vary depending on the type, particle size, moisture content and the like of the water absorbent resin.

When the surface crosslinking is executed, upon mixing of the water absorbent resin and the surface crosslinking agent, a hydrophilic organic solvent may be used in combination as needed. The hydrophilic organic solvent which can be used in this step may be, for example, any one of those illustrated above as the solvent which can be included in the aqueous fluid. Using amount of the hydrophilic organic solvent may fall within the range of preferably not more than 20 parts by weight, and more preferably not more than 10 parts by weight per 100 parts by weight of the solid content of the water absorbent resin, although it may vary depending on the type, particle size, moisture content and the like of the water absorbent resin.

When the surface crosslinking is executed, after mixing the water absorbent resin and the surface crosslinking agent, it is preferred that a heat treatment is further carried out to permit the crosslinking around the surface of the water absorbent resin. More specifically, for allowing the crosslinking agent to be reacted around the surface of the water absorbent resin, it is preferred to carry out the heat treatment in light of the reactivity of the crosslinking agent, simplicity of the apparatus for the production and productivity. Treatment temperature of the heat treatment is preferably not lower than 80°C although it may vary depending on the surface crosslinking agent. When the treatment temperature is lower than 80°C, time period required for the heat treatment may be so long that lowering of productivity may be caused, thereby failing to achieve homogenous surface crosslinking, and being liable to cause deterioration of absorption properties under a load and remaining of the surface crosslinking agent.

Although fine powder may be generated again due to damage caused during the process following the heat treatment in the surface crosslinking step, in such instances, the fine powder may be removed by further providing a classification step, which is similar to that described above, until the final product is obtained. Thus removed fine powder is preferably subjected to agglomeration together with the fine powder which had not been subjected to the surface crosslinking step (the fine powder obtained before the surface crosslinking step). In this step, amount of the fine powder classified following the surface crosslinking step is preferably not more than 5% by weight, and more preferably not more than 3% by weight of the amount of the fine powder which had not been subjected to the surface crosslinking step. When the amount of the fine powder classified following the surface crosslinking step is beyond 5% by weight, strength of the agglomerated particles is liable to be lowered, and the absorption capacity may be also declined.

To the water absorbent resin obtained by the method for production of the present invention may be added ad libitum, for example, a disinfectant, an antimicrobial agent, a flavor, any type of inorganic powder, a foaming agent, a pigment, a dye, a hydrophilic short fiber, a fertilizer, an oxidizing agent, a reducing agent, an aqueous salt and the like as needed, within the range not to impair the effect of the present invention (for example, not more than 30 parts by weight, further, not more than 10 parts by weight per 100 parts by weight of the water absorbent resin). Thus, various functions can be imparted.

According to the method for production of the present invention, a water absorbent resin achieving a balance between lowerings of the residual monomer and the fine powder, in other words, the water absorbent resin which is accompanied by less residual monomer and exhibits sharp particle size distribution can be obtained. Furthermore, a water absorbent resin with higher performance can be obtained by subjecting to the aforementioned surface crosslinking.

The water absorbent resin obtained by the method for production of the present invention has an absorption capacity for a physiological saline solution without load (GV) of preferably not less than 27 g/g, more preferably not less than 30 g/g, still more preferably not less than 33 g/g, and most preferably not less than 36 g/g. In general, when the absorption capacity without load is less than 20 g/g, favorable physical property may not be exhibited for use in diapers. In contrast, although the upper limit of the absorption capacity without load is not limited, approximately 60 g/g will be enough taking into consideration of possible rise of the costs due to difficulty which may be lead in the production.

The water absorbent resin obtained by the method for production of the present invention has an absorption capacity for a physiological saline solution under a load of 4.9 kPa (AAP) being preferably not less than 20 g/g, more preferably not less than 22 g/g, still more preferably not less than 24 g/g, and most preferably not less than 26 g/g. In general, when the absorption capacity under a load is less than 20 g/g, favorable physical property may not be exhibited for use in diapers. In contrast, although the upper limit of the absorption capacity under a load is not limited, approximately 35 g/g will be enough taking into consideration of possible rise of the costs due to difficulty which may be lead in the production.

According to the water absorbent resin obtained by the method for production of the present invention, the weight average particle diameter (D50) is controlled to fall within a comparatively narrow range of preferably 200 to 450 $\mu$m, more preferably 220 to 430 $\mu$m, and still more preferably 250 to 400 $\mu$m; content of the particles of less than 150 $\mu$m is controlled to be preferably 0 to 5% by weight, more preferably 0 to 3% by weight, and still more preferably 0 to 1% by weight; and logarithmic standard deviation ($\sigma\zeta$) of the particle size is controlled to be preferably 0.20 to 0.40, more preferably 0.20 to 0.38, and still more preferably 0.20 to 0.35. In the method for production of the present invention, the water absorbent resin having the specified particle size falling within the above range can be obtained by carrying out the classification step before subjecting to the surface crosslinking, or after subjecting to the surface crosslinking, as needed. Moreover, because the method for production of the present invention recycles the fine powder, a water absorbent resin can be obtained which is characterized by the content of the particles of less than 150 $\mu$m (fine powder) being small despite that the weight average particle diameter is comparatively small that is 200 to 450 $\mu$m, i.e., having both contradictory physical properties of small particle diameter and small amount of the fine powder in combination. When the weight average particle diameter (D50), content of the particles of less than 150 $\mu$m, and the logarithmic standard deviation ($\sigma\zeta$) of the particle size are out of the range described above, favorable physical property may not be exhibited for use in diapers.

The water absorbent resin obtained by the method for production of the present invention has a content of acrylic acid as determined by HPLC of preferably 0 to 1000 ppm, more preferably 0 to 500 ppm, still more preferably 0 to 200 ppm, and most preferably 0 to 100 ppm. In general, the content of acrylic acid being beyond 500 ppm is believed to be unpreferred because deodorizing action may be critically reduced.

Applications of the water absorbent resin obtained by the method for production of the present invention are not particularly limited, however, for example, use in absorbent cores and absorbing articles are preferred, and particularly, excellent performances may be achieved in highly dense diapers (those in which a large quantity of a water absorbent resin is used in a piece of diaper) which have conventionally involved problems of the odor.

Absorbent cores produced using the water absorbent resin obtained by the method for production of the present invention can be obtained by forming to give a sheet shape from, for example, the water absorbent resin and if necessary, a hydrophilic fiber. When the hydrophilic fiber is not used, the absorbent core can be obtained by fixing particulate water absorbing agent on paper or nonwoven fabric. It is desired that content of the water absorbent resin in such an absorbent core (core concentration) is high, which may be, for example, 30 to 100% by weight, preferably 40 to 100% by weight, more preferably 50 to 100% by weight, still more preferably 60 to 100% by weight, and particularly preferably 70 to 100% by weight. Also, such an absorbent core is desirably subjected to compression molding to have the density of 0.06 to 0.5 g/cc and grammage in the range of 0.01 to 0.2 g/cm$^2$. Illustrative examples of the fiber base material to be used include e.g., hydrophilic fibers such as ground wood pulp, cotton linters, cross-linked cellulosic fibers, rayon fibers, cotton fibers, wool fibers, acetate fibers, vinylon fibers, and the like, which are preferably airlied.

For example, the water absorbing agent according to the present invention obtained by the aforementioned method for production as one example has the amount of residual monomer determined by the method described later being not lower than 0 but not higher than 500 ppm. The amount of residual monomer is preferably not lower than 0 but not higher than 400 ppm, more preferably not lower than 0 but not higher than 300 ppm, more preferably not lower than 0 but not higher than 250 ppm, more preferably not lower than 0 but not higher than 200 ppm, still preferably not lower than 0 but not higher than 150 ppm, and particularly preferably not lower than 0 but not higher than 100 ppm. When the main component of the monomer used in the polymerization described above is acrylic acid and/or a salt thereof, content of unreacted acrylic acid and/or a salt thereof is not higher than 500 ppm. When the amount of residual monomer of the water absorbing agent of the present invention is beyond 500 ppm, abnormal odor may be developed after absorbing human urea in practical use as an absorbing article such as a diaper followed by swelling. Furthermore, taking into account of possibility of contact with the user's skin, problems in sanitary aspects may be also involved. Additionally, this amount is not preferred also because bad influence may be exerted on health of the worker due to scattered powder in manufacturing site of the absorbing article.

[Examples]

Hereinafter, the present invention will be explained more specifically by way of Examples, however, the present invention is not limited thereto. In the followings, unless particularly specified, "part by weight" will be merely denoted by "part", while "% by weight" will be denoted merely by "%".

Physical properties of the water absorbent resin obtained in Examples and Comparative Examples were measured as described below. Any of each measurement was conducted at a temperature in the range of 23 $\pm$ 2°C.

<Absorption Capacity without a Load (GV: Gel Volume)>

The water absorbent resin of about 0.2 g was accurately weighed (this weight being defined as "weight of water absorbent resin" in the following formula), homogenously put in a bag (60 mm x 60 mm) made of nonwoven fabric, and immersed in a 0.9% aqueous solution of sodium chloride (physiological saline solution). The bag was taken out 30 minutes later, and subjected to dewatering for 3 minutes at 250 x 9.81 m/s$^2$ (250 G) using a centrifuge, and thereafter, weight W1 (g) of the bag was measured. Also, similar operation was conducted without using any water absorbing agent, and the weight W0 (g) then was also measured. Thus, GV was calculated from the weights W1 and W0 according to the following formula.

$$GV \ (g/g) = [(W1 - W0)/ \text{ weight of water absorbent resin}]$$

$$- \ 1$$

<Amount of Residual Monomer>

Deionized water of 1000 g was weighed in a plastic vessel with a lid, and to the water was added 0.5 g of the water absorbent resin followed by stirring for 2 hrs. Using a filter paper, the swollen and gelled water absorbent resin was filtrated, and the resulting filtrate was analyzed on liquid chromatography. On the other hand, a monomer (acrylic acid) standard solution having a known concentration was similarly analyzed, and thus obtained calibration curve was used as an external standard. Accordingly, taking into account of the dilution factor of the filtrate, the amount of monomer in the water absorbent resin (amount of residual acrylic acid or salts thereof converted in terms of acrylic acid) was determined.

<Solid Content Rate of Polymer Gel or Agglomerated Gel>

A polymer gel or an agglomerated gel of about 5 g was accurately weighed in an aluminum cup having a diameter of 52 mm (this weight being defined as W0 (g)) : Subsequent to drying at 180°C in a windless dryer for 24 hrs, weight of the residue after drying was measured (this weight being defined as W1 (g)). Thus, solid content rate (%) was calculated from the weights W1 and W0 according to the following formula. However, when solid content rate (%) of the dried matter of the polymer gel which had been subjected to the drying step was determined, the aforementioned drying was conducted to reduce the time period of 3 hrs.

$$\text{Solid content rate } (\%) = (W1/W0) \ x \ 100$$

<Absorption Capacity under a Load (AAP: Absorbency against Pressure)>

According to the method disclosed in United States Patent No. 6071976, absorption capacity (value in 60 min) for a physiological saline solution under a load of 50 g/cm2 (4.9 kPa) was measured.

<Logarithmic Standard Deviation (σζ) of Particle Size and Weight Average Particle Diameter (D50)>

According to the method disclosed in WO2004/069936, sieve classification was conducted to determine logarithmic standard deviation (σζ) of the particle size and weight average particle diameter (D50).

[Production Example 1]

A polymer gel was produced using a belt type continuous polymerization apparatus equipped with an endless belt the surface of which being coated with a fluorocarbon resin (effective length: 3.8 m, width: 60 cm), a heating means for incubating the bottom face and the circumference of the endless belt at about 100°C, and a UV lamp for irradiating a light required for the polymerization reaction to the endless belt. More specifically, a monomer liquid was prepared through setting the flow rate of a 48.5% aqueous sodium hydroxide solution at 396 g/min, acrylic acid at 493 g/min, a 30% aqueous solution (I) of polyethylene glycol diacrylate (average molecular weight: 487) at 5.2 g/min, a solution (II) prepared by dissolving 1.0 part of 1-hydroxycyclohexylphenylketone and 1.1 parts of a 45% aqueous diethylenetriamine pentaacetate pentasodium solution in 97.9 parts of a 20% aqueous acrylic acid solution at 6.1 g/min, and water at 405 g/min, respectively, followed by continuously supplying to a mixer and mixing (this monomer liquid had a temperature of about 93°C being constant) ; a strip-shaped polymer gel was produced by continuously supplying a monomer mixture for polymerization (flow rate being 1333 g/min, monomer concentration in the monomer mixture being 45%, amount of sodium persulfate in the mixture corresponding to 0.091% per the weight of monomer component) on the belt traveling at a rate of 1 m/min, which was prepared while further adding a 2% aqueous sodiumm persulfate solution to the monomer liquid at a flow rate of 27.4 g/min, whereby executing polymerization on the belt; and the strip-shaped polymer gel thus produced was continuously detached from the belt. Then, the resulting strip-shaped polymer gel was continuously put into a screw type extruder (dice pore size being 13 mm) while separately adding water at a flow rate of 16.6 g/min to the inlet of the extruder to obtain a roughly crushed polymer gel (11). The polymer gel (11) had a solid content rate of 54%.

Next, 2000 g of the polymer gel (11) (quantity of solid content being 1080 g) was spread over a wire mesh, and subjected to hot-air drying using a ventilation flow batch-wise type dryer (manufactured by Satake Corporation "type 71-S6") at 180°C for 30 min. Then, the resulting dried matter was pulverized with a roll mill, and thereafter, using a JIS standard mesh sieve (Z8801) having mesh opening size of 850 $\mu$m, particles that pass through the mesh sieve were classified to obtain a particulate water absorbent resin (P1).

[Production Example 2]

A fine powdered water absorbent resin (P1') was obtained in a similar manner to Production Example 1 except that dried matter was pulverized with a roll mill having a narrower clearance between the rolls than that of the roll mill in Production Example 1 and a JIS standard mesh sieve having mesh opening size of 150 $\mu$m was used to classify the particles that pass through the mesh sieve.

Next, 300 g of the fine powdered water absorbent resin (P1') was put into a 5 L mortar mixer (manufactured by NISHI NIPPON SHIKENKI: KK) incubated in a water bath at 80°C, and thereto was charged at once 420 g of water as an aqueous fluid for agglomeration which had been previously heated to 80°C while rotating agitation blade of the mortar mixer at a high velocity of 60 HZ/100 V. Within 10 sec following charging, the fine powdered water absorbent resin (P1') was mixed with water, thereby turning into an agglomerated matter in a discrete state without tackiness. Agglomerated gel (21) having a particle diameter of 3 to 10 mm was obtained by recovering 1 min after the charging. The agglomerated gel (21) had a solid content rate of 42%.

Then, 2000 g of the agglomerated gel (21) obtained by repeating the agglomeration (quantity of solid content therein (corresponding to the quantity of the fine powdered water absorbent resin (P1')) being 833 g) was spread over a wire mesh, and subjected to hot-air drying using a ventilation flow batch-wise type dryer (manufactured by Satake Corporation "type 71-S6") at 180°C for 30 min. Then, pulverization was conducted with a roll mill, and thereafter, using a JIS standard mesh sieve (Z8801) having mesh opening size of 850 $\mu$m, particles that pass through the mesh sieve were classified to obtain a particulate water absorbent resin (P2).

[Production Example 3]

The polymer gel (11) obtained in Production Example 1 (quantity of solid content being 900 g) in an amount of 1667 g, and 333 g of the agglomerated gel (21) obtained in Production Example 2 (quantity of solid content therein (corresponding to the quantity of the fine powdered water absorbent resin (P1')) being 139 g) were briefly mixed and spread over a wire mesh, and subjected to hot-air drying using a ventilation flow batch-wise type dryer (manufactured by Satake Corporation "type 71-S6") at 180°C for 30 min. Then, pulverization was conducted with a roll mill, and thereafter, using a JIS standard mesh sieve (Z8801) having mesh opening size of 850 $\mu$m, particles that pass through the mesh sieve were classified to obtain a particulate water absorbent resin (P3).

[Example 1]

Agglomerated gel (22) having a particle diameter of 3 to 10 mm was obtained in a similar manner to Production Example 2 except that an aqueous fluid prepared by dissolving 0.069 g of sodium persulfate (accounting for 0.023% of the fine powdered water absorbent resin (P1'), and accounting for 25% of the amount of sodium persulfate used in the polymerization step (the amount of sodium persulfate used in polymerization step being 0.091% of the weight of the monomer component)) in 420g of water was used in place of 420 g of water which was used as the aqueous fluid for agglomeration in Production Example 2. The agglomerated gel (22) had a solid content rate of 42%.

Next, the polymer gel (11) obtained in Production Example 1 (quantity of solid content being 900 g) in an amount of 1667 g, and 333 g of the agglomerated gel (22) (quantity of solid content therein (corresponding to the quantity of the fine powdered water absorbent resin (P1')) being 139 g, and containing 0.032 g of sodium persulfate) were briefly mixed and spread over a wire mesh, and subjected to hot-air drying using a ventilation flow batch-wise type dryer (manufactured by Satake Corporation "type 71-S6") at 180°C for 30 min. Then, pulverization was conducted with a roll mill, and thereafter, using a JIS standard mesh sieve (Z8801) having mesh opening size of 850 $\mu$m, particles that pass through the mesh sieve were classified to obtain a particulate water absorbent resin (1). Of total amount of sodium persulfate included in the particulate water absorbent resin (1), the amount of added sodium persulfate except for that used in the polymerization step (accounting for 0.091% of the weight of monomer component) accounted for 0.0031% of total quantity of solid content of the water absorbent resin (1), which total quantity was added as an aqueous fluid upon obtaining the agglomerated gel.

[Comparative Example 1]

A strip-shaped polymer gel was produced in a similar manner to Production Example 1, and thus produced strip-shaped polymer gel was continuously detached from the belt. Then, the resulting strip-shaped polymer gel was continuously put into a screw type extruder (dice pore size being 13 mm) while separately adding a 0.13% aqueous sodium persulfate solution at a flow rate of 16.6 g/min (accounting for 0.0035% of the weight of monomer component in the monomer mixture for polymerization) to the inlet of the extruder to obtain a roughly crushed polymer gel (12). The polymer gel (12) had a solid content rate of 54%.

Next, the polymer gel (12) (quantity of solid content being 900 g, and containing 0.032 g of sodium persulfate as added to the polymer gel) in an amount of 1667 g, and 333 g of the agglomerated gel (21) (quantity of solid content therein (corresponding to the quantity of the fine powdered water absorbent resin (P1')) being 139 g) were combined, briefly mixed and spread over a wire mesh, and subjected to hot-air drying using a ventilation flow batch-wise type dryer (manufactured by Satake Corporation "type 71-S6") at 180°C for 30 min. Then, pulverization was conducted with a roll mill, and thereafter, using a JIS standard mesh sieve (Z8801) having mesh opening size of 850 $\mu$m, particles that pass through the mesh sieve were classified to obtain a particulate water absorbent resin (C1) for comparison. Of total amount of sodium persulfate included in the particulate water absorbent resin (C1), the amount of added sodium persulfate except for that used in the polymerization step (accounting for 0.091% of the weight of monomer component) accounted for 0.0031% of total quantity of solid content of the water absorbent resin (C1), which total quantity was added upon obtaining the polymer gel.

[Reference Example 1]

The polymer gel (12) obtained in Comparative Example 1 (quantity of solid content being 1080 g, and containing 0.038 g of sodium persulfate as added to the polymer gel) in an amount of 2000 g was spread over a wire mesh, and subjected to hot-air drying using a ventilation flow batch-wise type dryer (manufactured by Satake Corporation "type 71-S6") at 180°C for 30 min. Then, pulverization was conducted with a roll mill, and thereafter, using a JIS standard mesh sieve (Z8801) having mesh opening size of 850 $\mu$m, particles that pass through the mesh sieve were classified to obtain a particulate water absorbent resin (R1) as a reference. Of total amount of sodium persulfate included in the particulate water absorbent resin (R1), the amount of added sodium persulfate except for that used in the polymerization step (accounting for 0.091% of the weight of monomer component of the polymerization step) accounted for 0.0035% of total quantity of solid content of the water absorbent resin (R1), which total quantity was added upon obtaining the polymer gel.

[Example 2]

Agglomerated gel (23) having a particle diameter of 3 to 10 mm was obtained in a similar manner to Production Example 2 except that an aqueous fluid prepared by dissolving 0.69 g of sodium persulfate (accounting for 0.23% of the fine powdered water absorbent resin (P1')) in 420g of water was used in place of 420 g of water which was used as the aqueous fluid for agglomeration in Production Example 2. The agglomerated gel (23) had a solid content rate of 42%.

Next, a particulate water absorbent resin (2) was obtained in a similar manner to Example 1 except that 333 g of the agglomerated gel (23) (quantity of solid content therein (corresponding to the quantity of the fine powdered water absorbent resin (P1')) being 139 g, and containing 0.32 g of sodium persulfate) was used in stead of 333 g of the agglomerated gel (22) (quantity of solid content therein (corresponding to the quantity of the fine powdered water absorbent resin (P1')) being 139 g, and containing 0.032 g of sodium persulfate). Of total amount of sodium persulfate included in the particulate water absorbent resin (2), the amount of added sodium persulfate except for that used in the polymerization step (accounting for 0.091% of the weight of monomer component of the polymerization step) accounted for 0.031% of total quantity of solid content of the water absorbent resin (2), which total quantity was added as an aqueous fluid upon obtaining the agglomerated gel.

[Example 3]

Agglomerated gel (24) having a particle diameter of 3 to 10 mm was obtained in a similar manner to Production Example 2 except that an aqueous fluid prepared by dissolving 0.69 g of sodium bisulfite (accounting for 0.23% of the fine powdered water absorbent resin (P1')) in 420g of water was used in place of 420 g of water which was used as the aqueous fluid for agglomeration in Production Example 2. The agglomerated gel (24) had a solid content rate of 42%.

Next, a particulate water absorbent resin (3) was obtained in a similar manner to Example 1 except that 333 g of the agglomerated gel (24) (quantity of solid content therein (corresponding to the quantity of the fine powdered water absorbent resin (P1')) being 139 g, and containing 0.32 g of sodium bisulfite) was used in stead of 333 g of the agglomerated gel (22) (quantity of solid content therein (corresponding to the quantity of the fine powdered water absorbent resin (P1')) being 139 g, and containing 0.032 g of sodium persulfate). The amount of added sodium bisulfite accounted for 0.031% of total quantity of solid content of the water absorbent resin (3), which total quantity was added as an aqueous fluid upon obtaining the agglomerated gel.

[Comparative Example 2]

A strip-shaped polymer gel was produced in a similar manner to Production Example 1, and thus produced strip-shaped polymer gel was continuously detached from the belt. Then, the resulting strip-shaped polymer gel was continuously put into a screw type extruder (dice pore size being 13 mm) while separately adding a 1.3% aqueous sodium persulfate solution at a flow rate of 16.6 g/min (accounting for 0.035% of the weight of monomer component in the monomer mixture for polymerization) to the inlet of the extruder to obtain a roughly crushed polymer gel (13). The polymer gel (13) had a solid content rate of 54%.

Next, the polymer gel (13) (quantity of solid content being 900 g, and containing 0.32 g of sodium persulfate as added to the polymer gel) in an amount of 1667 g, and 333 g of the agglomerated gel (21) obtained in Production Example 2 (quantity of solid content therein (corresponding to the quantity of the fine powdered water absorbent resin (P1')) being 139 g) were combined, briefly mixed and spread over a wire mesh, and subjected to hot-air drying using a ventilation flow batch-wise type dryer (manufactured by Satake Corporation "type 71-S6") at 180°C for 30 min. Then, pulverization was conducted with a roll mill, and thereafter, using a JIS standard mesh sieve (Z8801) having mesh opening size of 850 $\mu$m, particles that pass through the mesh sieve were classified to obtain a particulate water absorbent resin (C2) for comparison. Of total amount of sodium persulfate included in the particulate water absorbent resin (C2), the amount of added sodium persulfate except for that used in the polymerization step (accounting for 0.091% of the weight of monomer component of the polymerization step) accounted for 0.031% of total quantity of solid content of the water absorbent resin (C2), which total quantity was added upon obtaining the polymer gel.

[Reference Example 2]

The polymer gel (13) obtained in Comparative Example 2 (quantity of solid content being 1080 g, and containing 0.38 g of sodium persulfate as added to the polymer gel) in an amount of 2000 g was spread over a wire mesh, and subjected to hot-air drying using a ventilation flow batch-wise type dryer (manufactured by Satake Corporation "type 71-S6") at 180°C for 30 min. Then, pulverization was conducted with a roll mill, and thereafter, using a JIS standard mesh sieve (Z8801) having mesh opening size of 850 $\mu$m, particles that pass through the mesh sieve were classified to obtain a particulate water absorbent resin (R2) as a reference. Of total amount of sodium persulfate included in the particulate water absorbent resin (R2), the amount of added sodium persulfate except for that used in the polymerization step (accounting for 0.091% of the weight of monomer component of the polymerization step) accounted for 0.035% of total quantity of solid content of the water absorbent resin (R2), which total quantity was added upon obtaining the polymer gel.

The absorption capacity without load (GV) and the amount of residual monomer of each water absorbent resin obtained in the foregoing Production Examples, Examples, Comparative Examples and Reference Examples are collectively shown in Table 1 and Table 2.

Table 1

| | | During drying | | Sodium persulfate *1 | | Absorption capacity without load (GV) (g/g) | Quantity of residual monomer (ppm) | Presence/ absence of recycling of pulverized powder |
| | | Polymer gel | Agglomerated gel | Amount (%) *2 | Subject of addition | | | |
|---|---|---|---|---|---|---|---|---|
| Production Example 1 | Water absorbent resin (P1) | (11) | Absent | 0 | - - | 42 | 350 | Absent |
| Production Example 2 | Water absorbent resin (P2) | Absent | (21) | 0 | - - | 36 | 430 | Pulverized powder alone was used |
| Production Example 3 | Water absorbent resin (P3) | (11) | (21) | 0 | - - | 41 | 530 | Present |
| Example 1 | Water absorbent resin (1) | (11) | (22) | 0,0031 | Agglomerated gel | 42 | 350 | Present |
| Comparative Example 1 | Water absorbent resin (C1) | (12) | (21) | 0,0031 | Polymer gel | 41 | 530 | Present |
| Reference 1 Example 1 | Water absorbent resin (R1) | (12) | Absent | 0,0035 | Polymer gel | 41 | 350 | Absent |
| Example 2 | Water absorbent resin (2) | (11) | (23) | 0,031 | Agglomerated gel | 42 | 220 | Present |
| Comparative Example 2 | Water absorbent resin (C2) | (13) | (21) | 0,031 | Polymer gel | 42 | 420 | Present |
| Reference Example 2 | Water absorbent resin (R2) | (13) | Absent | 0,036 | Polymer gel | 42 | 300 | Absent |
| *1: sodium persulfate added in addition to that used in the polymerization step<br>*2: amount represented by "%" per the quantity of solid content of the resulting water absorbent resin | | | | | | | | |

Table 2

| | | During drying | | Sodium bisulfite | | Absorption capacity without (GV) (g/g) | Quantity of residual load monomer (ppm) | Presence/ absence of recycling of pulverized powder |
|---|---|---|---|---|---|---|---|---|
| | | Polymer gel | Agglomerated gel | Amount(%) *3 | Subject of addition | | | |
| Example 3 | Water absorbent resin (3) | (11) | (24) | 0,031 | Agglomerated gel | 42 | 210 | Present |
| *3: amount represented by "%" per the quantity of solid content of the resulting water absorbent resin | | | | | | | | |

The followings are clear from Table 1 and Table 2.

That is, first, Production Example 1 shows the case in which recycling of the fine powder was not carried out but the polymer gel alone was dried (the amount of residual monomer being 350 ppm). In contrast, according to Production Example 2 in which only the agglomerated gel obtained by agglomerating the fine powder was dried, the residual monomer was increased to as much as 430 ppm. Next, when recycling of the fine powder was carried out through drying the polymer gel and the agglomerated gel together in Production Example 3, the amount of residual monomer was increased to 530 ppm, which is more than those in Production Example 1 and Production Example 2. In addition, it was revealed that this amount is dramatically greater than the amount of residual monomer (361 ppm) determined theoretically from the results obtained in Production Example 1 and Production Example 2, taking into account of the mixing ratio of the polymer gel and the agglomerated gel.

Example 1 shows the case in which sodium persulfate was added to the agglomerated gel in carrying out the recycling of the fine powder. In this case, the amount of residual monomer was 350 ppm, which is remarkably less than that in Production Example 3, clearly indicating similar results to those in the case of Production Example 1 in which the recycling of the fine powder was not carried out. Additionally, it is revealed that when the amount of sodium persulfate added to the agglomerated gel was increased in Example 2, the amount of residual monomer is further decreased than that in Production Example 1 in which the recycling of the fine powder was not carried out.

On the other hand, it is revealed that according to Comparative Example 1 in which sodium persulfate in the same amount as that in Example 1 was added to the polymer gel, the amount of residual monomer is 530 ppm, which is not lower compared to that in Production Example 3. Also, according to Comparative Example 2 in which the amount of sodium persulfate added to the polymer gel was increased, the amount of residual monomer becomes less than that in Production Example 3, however, the amount of residual monomer is more compared to the case of Production Example 1 in which the recycling of the fine powder was not carried out.

Example 3 shows the case in which sodium bisulfite was added to the agglomerated gel in carrying out the recycling of the fine powder. In this case, the amount of residual monomer is less than that in Production Example 3, indicating similar results to the case in which sodium persulfate was added to the agglomerated gel.

From the foregoings, it is clear that a balance between recycling of the fine powder and lowering of the residual monomer can be extremely effectively achieved, according to the present invention.

[Example 4]

A water absorbent resin (1') approximately entirety of which had a particle diameter of 850 μm to 150 μm was obtained by eliminating the fine powder of less than 150 μm from the water absorbent resin (1) obtained in Example 1 by further using a JIS standard mesh sieve having mesh opening size of 150 μm. Next, with 100 parts of the water absorbent resin (1'), was mixed a surface crosslinking agent comprising a mixed liquid of 0.34 parts of 1, 4-butanediol, 0.56 parts of propylene glycol and 3 parts of water, and thereafter, thus resulting mixture was subjected to a heat treatment at 210°C for 30 min to obtain water absorbent resin particles the surface of which is cross-linked. Measurement of physical properties of the water absorbent resin particle revealed that: the absorption capacity without load (GV) was 31 g/g; the absorption capacity under a load (AAP) was 26 g/g; the amount of residual monomer was 300 ppm; the weight average particle diameter (D50) was 420 μm; the content of particles of less than 150 μm (fine powder) was approximately 0% by weight; and the logarithmic standard deviation (σζ) of the particle size was 0.35.

The water absorbent resin and the method for production of a water absorbent resin according to the present invention can be suitably applied to production of absorbing articles such as sanitary goods including an absorbent core such as, for example, disposable diapers, sanitary napkins, incontinence pads and the like.

The foregoing explanations consistently show just examples, and various modifications can be made without departing from the principles of the present invention.

## Claims

1. A method for production of a water absorbent resin which comprises:

a step of obtaining a polymer gel having a water absorbing property,
a step of obtaining an agglomerated gel by adding an aqueous fluid containing at least one additive selected from the group consisting of thermal initiators, oxidizing agents and reducing agents to the fine powder which is obtained in the production of a water absorbent resin and has a weight average particle diameter falling within the range of 10 to 150 μm wherein the using amount of the aqueous fluid is not less than 25 parts by weight but not greater than 150 parts by weight per 100 parts by weight of the fine powder, and
a step of drying while allowing said agglomerated gel and said polymer gel to coexist wherein ratio (A/B) of the solid content rate A (%) of the agglomerated gel to the solid content rate B (%) of the polymer gel is not less than 1/3 but not greater than 3.

2. The method for production of a water absorbent resin according to claim 1 wherein said aqueous fluid is previously heated before addition to the fine powder.

3. The method for production of a water absorbent resin according to claim 1 or claim 2 wherein said additive comprises a persulfate.

4. The method for production of a water absorbent resin according to any of claims 1 to 3 wherein quantity of solid content in the agglomerated gel becomes not greater than 40% by weight of the quantity of solid content in the polymer gel, in said step of drying while allowing said agglomerated gel and said polymer gel to coexist.

5. A water absorbent resin which comprises a polymer gel having a water absorbing property and an agglomerated gel, wherein said agglomerated gel consists of fine powders having a weight average particle diameter falling within the range of from 10 to 150 $\mu$m; ratio (A/B) of the solid content rate A (%) of said agglomerated gel to the solid content rate B (%) of the polymer gel is not less than 1/3 but not greater than 3, and the amount of residual monomer is not lower than 0 but not higher than 500 ppm.

**Patentansprüche**

1. Verfahren zur Herstellung eines wasserabsorbierenden Harzes, das umfasst:

einen Schritt des Erhaltens eines Polymergels, das eine wasserabsorbierende Eigenschaft aufweist, einen Schritt des Erhaltens eines agglomerierten Gels durch Hinzugeben eines wässrigen Fluids, das mindestens ein Additiv ausgewählt aus der Gruppe aus thermischen Initiatoren, Oxidationsmitteln und Reduktionsmitteln enthält, zu dem Feinpulver, das bei der Herstellung eines wasserabsorbierenden Harzes erhalten wird und einen Gewichtsmittel-Teilchendurchmesser aufweist, der in den Bereich von 10 bis 150 $\mu$m fällt, wobei die Verwendungsmenge des wässrigen Fluids nicht weniger als 25 Gewichtsteile, aber nicht mehr als 150 Gewichtsteile pro 100 Gewichtsteile des Feinpulvers ist, und einen Schritt des Trocknens, während man das agglomerierte Gel und das Polymergel koexistieren läßt, wobei das Verhältnis (A/B) des Feststoffgehaltanteils A (%) des agglomerierten Gels zu dem Feststoffgehaltanteil B (%) des Polymergels nicht weniger als 1/3, aber nicht größer als 3 ist.

2. Verfahren zur Herstellung eines wasserabsorbierenden Harzes gemäß Anspruch 1, wobei das wässrige Fluid vor der Zugabe zu dem Feinpulver vorher erwärmt wird.

3. Verfahren zur Herstellung eines wasserabsorbierenden Harzes gemäß Anspruch 1 oder Anspruch 2, wobei das Additiv ein Persulfat umfasst.

4. Verfahren zur Herstellung eines wasserabsorbierenden Harzes gemäß einem der Ansprüche 1 bis 3, wobei die Menge des Feststoffgehalts in dem agglomerierten Gel nicht größer als 40 Gewichts-% der Menge des Feststoffgehalts in dem Polymergel wird, in dem Schritt des Trocknens, während man das agglomerierte Gel und das Polymergel koexistieren läßt.

5. Wasserabsorbierendes Harz, das ein Polymergel umfasst, das eine wasserabsorbiernde Eigenschaft aufweist, und ein agglomeriertes Gel, wobei das agglomerierte Gel aus Feinpulvern besteht, die einen Gewichtsmittel-Teilchendurchmesser aufweisen, der in den Bereich von 10 bis 150 $\mu$m fällt; das Verhältnis (A/B) des Feststoffgehaltanteils A (%) des agglomerierten Gels zu dem Feststoffgehaltanteil B (%) des Polymergels nicht weniger als 1/3, aber nicht größer als 3 ist und die Menge an restlichem Monomer nicht niedriger als 0, aber nicht höher als 500 ppm ist.

**Revendications**

1. Procédé de production d'une résine absorbant l'eau qui comprend :

une étape d'obtention d'un gel de polymère ayant la propriété d'absorber l'eau,

une étape d'obtention d'un gel aggloméré par addition d'un liquide aqueux contenant au moins un additif choisi parmi le groupe constitué d'initiateurs thermiques, d'agents oxydants et d'agents réducteurs à une poudre fine qui est obtenue dans la production d'une résine absorbant l'eau et a un diamètre de particule moyen en poids tombant dans la plage de 10 à 150 $\mu$m dans lequel la quantité utilisée de liquide aqueux n'est pas inférieure à 25 parties en poids mais n'est pas supérieure à 150 parties en poids par 100 parties en poids de poudre fine, et une étape de séchage tout en permettant audit gel aggloméré et audit gel de polymère de coexister dans lequel le rapport (A/B) de la teneur en matière solide totale A (%) du gel aggloméré à la teneur en matière solide totale B (%) du gel de polymère n'est pas inférieur à 1/3 mais n'est pas supérieur à 3.

2.  Procédé de production d'une résine absorbant l'eau selon la revendication 1 dans lequel ledit liquide aqueux est préalablement chauffé avant l'addition à la poudre fine.

3.  Procédé de production d'une résine absorbant l'eau selon la revendication 1 ou la revendication 2 dans lequel ledit additif comprend un persulfate.

4.  Procédé de production d'une résine absorbant l'eau selon l'une quelconque des revendications 1 à 3 dans lequel la teneur en matière solide totale dans le gel aggloméré ne devient pas supérieure à 40% en poids de la teneur en matière solide totale dans le gel de polymère, dans ladite étape de séchage tout en permettant audit gel aggloméré et audit gel de polymère de coexister.

5.  Résine absorbant l'eau qui comprend un gel de polymère ayant la propriété d'absorber l'eau et un gel aggloméré, dans lequel ledit gel aggloméré est constitué de poudres fines ayant un diamètre de particule moyen en poids tombant dans la plage de 10 à 150 $\mu$m ; le rapport (A/B) de la teneur en matière solide totale A (%) dudit gel aggloméré à la teneur en matière solide totale B (%) du gel de polymère n'est pas inférieur à 1/3 mais n'est pas supérieur à 3, et la quantité de monomère résiduel n'est pas inférieure à 0 mais n'est pas supérieure à 500 ppm.

**EP 1 690 887 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5342899 A
- US 4970267 A
- US 4950692 A
- US 6458921 B
- JP 63007203 B
- JP 2004517179 A
- US 0068057 A1
- US 6071976 A
- WO 2004069936 A